# EUROPEAN PATENT SPECIFICATION

(11) **EP 3 024 437 B1**
(45) Date of publication and mention of the grant of the patent: **10.10.2018**
(21) Application number: 14750872.5
(22) Date of filing: 24.07.2014
(51) Int. Cl.: A61K 8/64, A61Q 11/00

(54) **ORAL CARE COMPOSITION COMPRISING ESTERIFIED PROLAMINE**
MUNDPFLEGEZUSAMMENSETZUNG ENTHALTEND VERESTERTE PROLAMIN
COMPOSITION DE SOIN BUCCAL CONTENANT UNE PROLAMINE ESTÉRIFIÉ

(30) Priority: 25.07.2013 EP 13003726
(43) Date of publication of application: 01.06.2016
(73) Proprietor: Cargill, Incorporated, Wayzata, MN 55391 (US)
(72) Inventor: DE SADELEER, Jos Willy Ghislain Corneel, B-3220 Holsbeek (BE)
(74) Representative: Kiadi Matsuela, Dela
(86) International application number: PCT/US2014/048021
(87) International publication number: WO 2015/013514

(56) References cited:
- WO-A1-2013/070184
- WO-A2-2013/010119
- JP-A- H 029 807
- DATABASE WPI Week 200145 Thomson Scientific, London, GB; AN 2001-420672 XP002717814, & JP 2001 097836 A (OSAKA KAGAKU GOKIN KK) 10 April 2001 (2001-04-10) & JP 2001 097836 A (OSAKA KAGAKU GOKIN KK) 10 April 2001 (2001-04-10)
- DATABASE WPI Week 201348 Thomson Scientific, London, GB; AN 2013-H53454 XP002717815, & CN 102 846 488 A (JIANGSU LONGLIQI GROUP CO LTD) 2 January 2013 (2013-01-02)
- DATABASE WPI Week 199708 Thomson Scientific, London, GB; AN 1997-077986 XP002717816, & CN 1 077 884 A (YIN C) 3 November 1993 (1993-11-03)
- DATABASE WPI Week 200369 Thomson Scientific, London, GB; AN 2003-722704 XP002717817, & CN 1 403 072 A (LI C) 19 March 2003 (2003-03-19)
- DATABASE WPI Week 199736 Thomson Scientific, London, GB; AN 1997-386169 XP002717818, & CN 1 111 123 A (XINJIN BIOENGINEERING INST CHENGDU) 8 November 1995 (1995-11-08)
- DATABASE WPI Week 200377 Thomson Scientific, London, GB; AN 2003-814301 XP002717819, & CN 1 435 164 A (WEI Y) 13 August 2003 (2003-08-13)

## Description

### CROSS-REFERENCE TO RELATED APPLICATIONS

This application claims the benefit of European Application Serial No. 13003726.0 filed 25 July 2013.

### FIELD OF THE INVENTION

The present invention relates to oral care composition comprising a film-forming composition.

### BACKGROUND

Oral care compositions available on the market today, including for example mouthwashes and toothpaste, claim to have many beneficial effects. However, many of these are either only very temporary (e.g. breath freshness from mouthwash) or very ineffective (e.g. so-called whitening toothpastes). The cause of this can be linked directly to the short contact time between the composition and the mouth.

One option for addressing these drawbacks is to increase contact time. This can be achieved, for example, by providing oral care compositions in the form of thick pastes or with dental trays that have to remain in place for extended periods (e.g. for several hours or over-night). These solutions, however, are often considered unpleasant or impractical by the end user.

Another option is to increase the strength of the composition, i.e. by increasing the concentration of any active ingredients. Unfortunately, as these are often controlled or potentially hazardous in high concentrations (e.g. hydrogen peroxide which is used as a whitening agent), the compositions become unsuitable for domestic or unsupervised use and have to be administered by specialists such as dentists or dental hygienists. This makes the treatments both impractical and expensive as well as potentially harmful.

There is thus a need to provide oral care compositions that can be used as part of a normal, daily routine but which bring more long-term benefits or are more effective than products that are currently available on the market.

CN102846488 discloses a tooth beautifying liquid which forms a film on the teeth for protecting them.

WO2013070184 discloses dental film-forming compositions comprising polymeric substances, and optionally further whitening materials or other active agents.

### STATEMENTS OF THE INVENTION

According to one aspect of the present invention, there is provided an oral care composition comprising a film-forming composition, wherein the oral care composition is in the form of a dentifrice, an abrasive gel, a non-abrasive gels, a mouthwash, a mouth-rinse, a gargle, an irrigating solution, a mouth-spray or a composition intended for sucking or chewing, and wherein the film-forming composition comprises a vegetable protein, wherein the protein comprises a prolamine protein, wherein the prolamine protein comprises esterified prolamine protein.

According to related aspect of the present invention, there is provided a process comprising the steps of: providing an esterified prolamine protein; solubilising the prolamine protein in a solvent composition; and mixing the solubilized prolamine protein with one or more additional ingredients to form an oral care composition.

### DETAILED DESCRIPTION

The present invention provides an oral care composition comprising a film-forming composition, wherein the oral care composition is in the form of a dentifrice, an abrasive gel, a non-abrasive gels, a mouthwash, a mouth-rinse, a gargle, an irrigating solution, a mouth-spray or a composition intended for sucking or chewing, and wherein the film-forming composition comprises a vegetable protein, wherein the protein comprises a prolamine protein, wherein the prolamine protein comprises esterified prolamine protein.

The term "film-forming" as used herein refers to compositions that are capable of forming a film in an aqueous environment. In particular, the film-forming composition of the present invention will be capable of forming a film in the mouth, preferably over the teeth. The film will preferably be durable, i.e. remaining substantially intact for at least 4 hours, more preferably at least 6 hours, more preferably at least 8 hours, more preferably at least 10 hours, more preferably at least 12 hours. In one aspect, it will be resistant to non-abrasive wear (i.e. it
will not be removed by eating). It will also advantageously be grease-resistant and will preferably not deteriorate in the presence of alkali or acidic foodstuffs or beverages. Ideally, the film will remain substantially intact until deliberately removed. Intentional removal could be achieved, in the most straightforward form, simply by brushing with a toothbrush or other mechanical means. Alternatively, a special composition could be provided that would cause the film to deteriorate (e.g. by enzymatic action) or to dissolve (e.g. with a solvent).

Prolamine proteins are a group of plant storage proteins found in the seeds of cereal grains. They are characterised by a high proline and a high glutamine content. Examples of suitable prolamine proteins for use in the present invention include gliadin, hordein, secalin, zein, kafirin, avenin and mixtures of two or more thereof. Preferably, the protein used in the present invention will comprise zein. More preferably, it will consist essentially of zein.

Zein is a prolamine protein found in corn. It is usually provided in a form which exhibits yellow colouring due to the presence of pigment impurities including xanthophylls - such as zeaxanthin - and beta-carotene. Although zein can be used in this form in the present invention, it will preferably have a reduced xanthophyll and/or beta-carotene content. Preferably the xanthophyll and/or beta-carotene content is less than 200µg/g, preferably less than 150µg/g, more preferably less than 100µg/g, even more preferably less than 80µg/g, yet even more preferably less than 50µg/g, yet even more preferably less than 25µg/g, yet even more preferably less than 20µg/g, yet even more preferably less than 15µg/g of zein. Most preferably, it will be substantially free of xanthophylls and/or beta-carotenes. This can be achieved either by selecting zein from sources having a naturally lower pigment content (e.g. white corn) or by decolorizing the zein. Xanthophyll and beta-carotene content can be reduced, for example, by solvent extraction, treatment with active carbon, filtration, ultrafiltration, chromo-separation or by any other known means (as described, for instance, in Sessa et al. "Improved method for decolorizing corn zein" Industrial Crops and products 18(2003) 55-65). Thus, the zein used in the present invention will preferably be partly or fully decolorized zein.

The proteins used in the present invention are modified by esterification. Modification of a prolamine protein with octenyl succinate can be used to modulate its hydrophobicity. In one aspect, it may be desirable to use a prolamine protein with increased hydrophobicity as this would contribute to greater film strength and durability. In another aspect, it may be desirable to reduce the protein's hydrophobicity so that the film would naturally dissolve over time, in the mouth, so that it would not need to be deliberately removed (e.g. by brushing).

Advantageously, the oral care composition of the present invention will comprise 5-80%, preferably 10 to 70%, more preferably 15 to 60%, even more preferably 25 to 50%, yet even more preferably 30 to 50%, yet even more preferably 35 to 50%, yet even more preferably 40 to 50% protein, preferably prolamine protein, by weight based on total weight of the composition. The exact content will of course depend on the form of the oral care composition and will be readily determined by a skilled person. A paste or gel may comprise higher levels than a mouthwash, for instance. In any event, sufficient protein should be available to provide film-forming properties.

The oral care composition may further comprise a plasticiser. The term "plasticiser" as used herein will refer to any compound or composition capable of plasticising a protein, preferably capable of plasticising a prolamine protein. Examples of such plasticisers include, but are not limited to, oleic acids or other suitable fatty acids (preferably longer chain fatty acids), polyols such as glycerol or sorbitol, fatty alcohols, alkane carboxylic compounds, and mixtures thereof. These plasticisers may simply be mixed with the film-forming composition. Alternatively, they may be mixed and then reacted to cause the plasticiser to be bound to the film-forming composition. For example, zein and oleic acid can be esterified to produce a plasticised protein.

The oral care composition will preferably include a solvent composition in which the protein is soluble. In the case of prolamine proteins, this would include a composition of organic solvents such as ethanol, isopropanol, glycerol, ethyl lactate, propylene glycol, sorbitol and mixtures thereof. Preferably, the oral care composition will be solvent based. Such compositions include, by way of illustration only, mouthwashes and mouth-rises, gargles, irrigating solutions, and mouth-sprays. Alternatively, the oral care composition may be in the form of a gel or paste and will then preferably include a structuring agent, thickener or filling agent.

The oral care composition may further comprise one or more additional ingredients. These may be selected, for example, from optical brightening agents, whitening agents, anti-plaque agents, anti-cariogenic agents, anti-bacterial agents, breath-freshening agents, flavouring agents, pigments, and mixtures of two or more thereof. Examples of such additional ingredients will be readily apparent and available to the skilled person and will preferably be selected such that they do not cause significant disruption, degradation or denaturing of the film forming composition.

According to one particularly preferred aspect of the present invention, the oral care composition would combine a film forming composition and an optical brightening and/or whitening agent in the form, for instance, of a mouthwash or mouth-rinse. In using the mouthwash or mouth-rise, a film incorporating brightening and/or whitening agents would be formed over the end user's teeth, ensuring prolonged contact and prolonged effect. Alternatively, the film-forming composition can be used to produce an oral care composition with a slow or delayed release of certain active ingredients (such as breath-freshening agents, anti-bacterial or anti-cariogenic agents, flavouring agents and so on). Thus, the present invention also provides a slow-release or delayed-release oral care composition comprising a film-forming composition and one or more active ingredients.

The present invention further provides a process of preparing an oral care composition comprising adding a film-forming composition into an oral care composition. Preferably, the process will include the steps of:
- providing an esterified prolamine protein
- solubilising the prolamine protein in a solvent composition; and
- mixing the solubilized prolamine protein with one or more additional ingredients to form an oral care composition,
wherein the terms used herein take on the meanings set forth above.

In one aspect, the process would include the step, prior to solubilisation, of treating the prolamine protein to reduce xanthophyll and beta-carotene content. This treatment step could include solvent extraction, treatment with active carbon, filtration, ultrafiltration, chrome-separation and any other available means as noted above.

The process may also include a plasticizing step whereby a plasticizer is added to the composition. The plasticizer may be added directly to the oral care composition or it may be pre-mixed with the film-forming composition. Plasticizers may also be pre-reacted with the film-forming composition to cause binding. For example, the film-forming composition and the plasticizer may be chemically or enzymatically reacted. In one aspect, the film-forming composition - preferably comprising or consisting of a prolamine protein - and the plasticizer may be esterified.

Oral care composition obtainable according to the above process, and their use to increase the efficiency or duration of oral care treatments, are also part of the present invention.

The present invention further relates to the use of esterified zein to increase the duration of film forming composition in oral care composition. The use of esterified zein allows the production of film that will preferably be durable, i.e. remaining substantially intact for at least 4 hours, more preferably at least 6 hours, more preferably at least 8 hours, more preferably at least 10 hours, more preferably at least 12 hours in the mouth.

## Claims

1. An oral care composition comprising a film-forming composition, wherein the oral care composition is in the form of a dentifrice, an abrasive gel, a non-abrasive gels, a mouthwash, a mouth-rinse, a gargle, an irrigating solution, a mouth-spray or a composition intended for sucking or chewing, and wherein the film-forming composition comprises a vegetable protein, wherein the protein comprises a prolamine protein, wherein the prolamine protein comprises esterified prolamine protein.

2. An oral care composition according to claim 1, wherein the prolamine protein comprises a protein is selected from gliadin, hordein, secalin, zein, kafirin, avenin and mixtures of two or more thereof.

3. An oral care composition according to claim 1, wherein the prolamine protein consists essentially of zein.

4. An oral care composition according to claim 3, wherein the zein has a reduced xanthophyll and/or beta-carotene content.

5. An oral care composition according to claim 3 or claim 4 which is substantially free of xanthophyll and/or beta-carotene.

6. An oral care composition according to claim 1, wherein the esterified prolamine protein is esterified with octenyl succinate.

7. An oral care composition according to any one of claims 1 to 6, comprising 5-80% prolamine protein.

8. An oral care composition, according to any one of the preceding claims, further comprising one or more additional ingredients selected from: an optical brightening agent, a whitening agent, an anti-plaque agent, an anti-cariogenic agent, an anti-bacterial agent, a breath-freshening agent, a flavouring agent, a pigment, or a mixture of two or more thereof.

9. An oral care composition according to any one of the preceding claims which is selected from a mouthwash, a mouth-rinse or a mouth-spray.

10. A process of producing an oral care composition comprising:
- providing a prolamine protein, wherein the prolamine protein comprises esterified prolamine protein
- solubilising the prolamine protein in a solvent composition; and
- mixing the solubilized prolamine protein with one or more additional ingredients to form an oral care composition,
wherein the oral care composition is in the form of a dentifrice, an abrasive gel, a non-abrasive gels, a mouthwash, a mouth-rinse, a gargle, an irrigating solution, a mouth-spray or a composition intended for sucking or chewing.

11. A process according to claim 10, which further comprises the step, prior to solubilisation, of treating the prolamine protein to reduce xanthophyll and beta-carotene content.

## Patentansprüche

1. Mundpflegezusammensetzung, die eine filmbildende Zusammensetzung umfasst, wobei die Mundpflegezusammensetzung in Form einer Zahnpasta, eines abrasiven Gels, eines nicht abrasiven Gels, einer Mundspülung, eines Mundwassers, eines Gurgelwassers, einer Spüllösung, eines Mundsprays oder einer zum Saugen oder Kauen bestimmten Zusammensetzung ist und wobei die filmbildende Zusammensetzung ein pflanzliches Protein umfasst, wobei das Protein ein Prolamin-Protein umfasst, wobei das Prolamin-Protein verestertes Prolamin-Protein umfasst.

2. Mundpflegezusammensetzung nach Anspruch 1, wobei das Prolamin-Protein ein Protein umfasst, das aus Gliadin, Hordein, Secalin, Zein, Kafirin, Avenin und Gemischen aus zwei oder mehr davon ausgewählt ist.

3. Mundpflegezusammensetzung nach Anspruch 1, wobei das Prolamin-Protein im Wesentlichen aus Zein besteht.

4. Mundpflegezusammensetzung nach Anspruch 3, wobei das Zein einen reduzierten Xanthophyll- und/oder Beta-Carotingehalt aufweist.

5. Mundpflegezusammensetzung nach Anspruch 3 oder Anspruch 4, die im Wesentlichen frei von Xanthophyll- und/oder Beta-Carotin ist.

6. Mundpflegezusammensetzung nach Anspruch 1, wobei das veresterte Prolamin-Protein mit Octenylsuccinat verestert ist.

7. Mundpflegezusammensetzung nach einem der Ansprüche 1 bis 6, die 5-80% Prolamin-Protein umfasst.

8. Mundpflegezusammensetzung nach einem der vorstehenden Ansprüche, weiter umfassend einen oder mehrere zusätzlich Inhaltsstoffe, ausgewählt aus: einem optischen Aufhellungsmittel, einem weißmachenden Mittel, einem Anti-Plaque-Mittel, einem antikariogenen Mittel, einem antibakteriellen Mittel, einem Atemerfrischungsmittel, einem Aromastoff, einem Pigment oder einem Gemisch aus zwei oder mehr davon.

9. Mundpflegezusammensetzung nach einem der vorstehenden Ansprüche, die ausgewählt ist aus einer Mundspülung, einem Mundwasser oder einem Mundspray.

10. Verfahren zur Herstellung einer Mundpflegezusammensetzung umfassend:
- Bereitstellen eines Prolamin-Proteins, wobei das Prolamin-Protein verestertes Prolamin-Protein umfasst
- Auflösen des Prolamin-Proteins in einer Lösungsmittelzusammensetzung; und
- Mischen des aufgelösten Prolamin-Proteins mit einem oder mehreren Inhaltsstoffen, um eine Mundpflegezusammensetzung zu bilden,
wobei die Mundpflegezusammensetzung in der Form einer Zahnpasta, eines abrasiven Gels, eines nicht abrasiven Gels, einer Mundspülung, eines Mundwassers, eines Gurgelwassers, einer Spüllösung, eines Mundsprays oder einer zum Saugen oder Kauen bestimmten Zusammensetzung ist.

11. Verfahren nach Anspruch 10, das weiter den Schritt des Behandelns des Prolamin-Proteins vor dem Auflösen zum Reduzieren des Xanthophyll- und Beta-Carotingehalts umfasst.

## Revendications

1. Composition de soin buccal comprenant une composition filmogène, dans laquelle la composition de soin buccal est sous forme d'un dentifrice, d'un gel abrasif, d'un gel non-abrasif, d'un bain de bouche, d'un rince-bouche, d'une eau dentaire, d'une solution d'irrigation, d'un spray buccal ou d'une composition destinée à être sucée ou mâchée, et dans laquelle la composition filmogène comprend une protéine végétale, dans laquelle la protéine comprend une protéine prolamine, dans laquelle la protéine prolamine comprend une protéine prolamine estérifiée.

2. Composition de soin buccal selon la revendication 1, dans laquelle la protéine prolamine comprend une protéine sélectionnée parmi de la gliadine, de l'hordéine, de la sécaline, de la zéine, de la kafirine, de l'avénine et des mélanges de deux ou plusieurs de celles-ci.

3. Composition de soin buccal selon la revendication 1, dans laquelle la protéine prolamine se compose essentiellement de zéine.

4. Composition de soin buccal selon la revendication 3, dans laquelle la zéine présente une teneur réduite en xanthophylle et/ou bêta-carotène.

5. Composition de soin buccal selon la revendication 3 ou la revendication 4 qui est sensiblement sans xanthophylle et/ou bêta-carotène.

6. Composition de soin buccal selon la revendication 1, dans laquelle la protéine prolamine estérifiée est estérifiée avec de l'octényle succinate.

7. Composition de soin buccal selon l'une quelconque des revendications 1 à 6, comprenant 5 à 80 % de protéine prolamine.

8. Composition de soin buccal, selon l'une quelconque des revendications précédentes, comprenant en outre un ou plusieurs ingrédients supplémentaires sélectionnés parmi : un agent d'azurage optique, un agent de blanchissement, un agent anti-tartre, un agent anti-carie, un agent antibactérien, un agent rafraîchisseur d'haleine, un agent aromatisant, un pigment, ou un mélange de deux ou de plusieurs de ceux-ci.

9. Composition de soin buccal selon l'une quelconque des revendications précédentes qui est sélectionnée parmi un bain de bouche, un rince-bouche ou un spray buccal.

10. Procédé de production d'une composition de soin buccal comprenant :
- une fourniture d'une protéine prolamine, dans lequel la protéine prolamine comprend une protéine prolamine estérifiée
- une solubilisation de la protéine prolamine dans une composition de solvant ; et
- un mélange de la protéine prolamine solubilisée avec un ou plusieurs ingrédients supplémentaires pour former une composition de soin buccal,
dans lequel la composition de soin buccal est sous forme d'un dentifrice, d'un gel abrasif, d'un gel non-abrasif, d'un bain de bouche, d'un rince-bouche, d'une eau dentaire, d'une solution d'irrigation, d'un spray buccal ou d'une composition destinée à être sucée ou mâchée

11. Procédé selon la revendication 10, qui comprend en outre l'étape, avant une solubilisation, de traitement de la protéine prolamine pour réduire une teneur en xanthophylle et en bêta-carotène.
